(19) Europäisches Patentamt
European Patent Office
Office européen des brevets

(11) EP 1 819 659 B1

(12) **EUROPÄISCHE PATENTSCHRIFT**

(45) Veröffentlichungstag und Bekanntmachung des Hinweises auf die Patenterteilung:
**28.05.2008 Patentblatt 2008/22**

(21) Anmeldenummer: **05817997.9**

(22) Anmeldetag: **24.11.2005**

(51) Int Cl.:
***C07C 51/60*** *(2006.01)* ***C07C 63/22*** *(2006.01)*

(86) Internationale Anmeldenummer:
**PCT/EP2005/012564**

(87) Internationale Veröffentlichungsnummer:
**WO 2006/056436 (01.06.2006 Gazette 2006/22)**

(54) **VERFAHREN ZUR HERSTELLUNG VON PHTHALSAUREDICHLORID**

METHOD FOR THE PRODUCTION OF PHTHALIC DICHLORIDE

PROCEDE POUR PRODUIRE DU CHLORURE DE PHTALOYLE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **26.11.2004 DE 102004057146**

(43) Veröffentlichungstag der Anmeldung:
**22.08.2007 Patentblatt 2007/34**

(73) Patentinhaber: **BASF SE**
**67056 Ludwigshafen (DE)**

(72) Erfinder:
- **ROHDE, Thorsten**
  **68305 Mannheim (DE)**
- **HENKELMANN, Jochem**
  **68165 Mannheim (DE)**

(56) Entgegenhaltungen:
**EP-A- 0 050 775** **GB-A- 1 464 463**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von Phthalsäuredichlorid durch Umsetzung von Phthalsäureanhydrid mit einem Chlorierungsmittel (I), ausgewählt aus der Gruppe Thionylchlorid und Phosgen, in Gegenwart eines Katalysators bei einer Temperatur von 80 bis 200°C und einem Druck von 0,01 bis 10 MPa abs.

Phthalsäuredichlorid ist ein wichtiges Ausgangsprodukt in der Herstellung von Weichmachern und Kunstharzen. Des Weiteren wird Phthalsäuredichlorid auch als Synthesebaustein in der Herstellung von Wirkstoffen, wie beispielsweise von Pharmazeutika und Pflanzenschutzmitteln eingesetzt.

Phthalsäuredichlorid wird im Allgemeinen durch Umsetzung von Phthalsäureanhydrid mit geeigneten Chlorierungsmitteln hergestellt.

O. Gräbe in Liebigs Ann., 1887, Seite 318 bis 337 (Seite 329 Fußnote) und S. Wolfe et al. in Canadian Journal of Chemistry 48, 1970, Seite 3566 bis 3571 offenbaren die Herstellung von Phthalsäuredichlorid durch Umsetzung von Phthalsäureanhydrid mit Phosphor(V)-chlorid. Nachteilig an diesem Verfahren ist die nur geringe Ausbeute von 54 % gemäß S. Wolfe et al., Canadian Journal of Chemistry 48, 1970, Seite 3570 sowie die Bildung stöchiometrischer Mengen an Phosphoroxidtrichlorid als Koppelprodukt, welches vom Wertprodukt Phthalsäuredichlorid abzutrennen und aufwändig zu entsorgen ist. Ferner ist Phosphor(V)-chlorid unter Normalbedingungen ein Feststoff und daher technisch schwieriger zu handhaben als beispielsweise eine Flüssigkeit oder ein Gas.

GB 1 464 463 offenbart ein Verfahren zur Herstellung von Carbonsäurechloriden durch Umsetzung von Carbonsäuren oder deren Anhydriden mit Phosgen in Gegenwart von trisubstituierten Phosphinoxiden als katalysatoren.

US 2,051,096 beschreibt die Herstellung von Phthalsäuredichlorid durch Umsetzung von Phthalsäureanhydrid mit Trichlormethan beziehungsweise Tetrachlormethan in Gegenwart von Zinkchlorid. Dieses Verfahren weist eine Reihe von Nachteilen auf. So gilt der Einsatz chlorierter Kohlenwasserstoffe aus heutiger umweltpolitischer Sicht als eher problematisch. Zudem erfordert das genannte Verfahren eine hohe Reaktionstemperatur von etwa 220 bis 300°C.

Des Weiteren ist aufgrund des Einsatzes einer metall-kationischen Lewissäure (Zinkchlorid) die anschließende Aufarbeitung des erhaltenen Reaktionsgemisches aufwändig, da nach der Abdestillation des Wertprodukts Phthalsäuredichlorid im Sumpf ein Gemisch übrig bleibt, welches die eingesetzte Lewissäure und die gebildeten organischen Nebenprodukte enthält. Dieses kann nicht einfach entsorgt werden, sondern ist in einem weiteren, aufwändigen Schritt in die metall-katiönische Lewissäure und die organischen Nebenprodukte aufzutrennen. Die metall-kationische Lewissäure und die organischen Nebenprodukte sind dann getrennt zu entsorgen.

DE-A 20 36 171 lehrt die Herstellung von Phthalsäuredichlorid durch Umsetzung von Phthalsäureanhydrid mit Trichlormethylisocyanatdichlorid in Gegenwart einer Lewissäure, insbesondere Zinkchlorid, Eisen(III)-chlorid oder Aluminiumchlorid. Nachteilig an diesem Verfahren ist die Bildung stöchiometrischer Mengen an Chlorcarbonylisocyaniddichlorid und Fumarsäuredichlorid als Koppelprodukte, welche vom Wertprodukt Phthalsäuredichlorid abzutrennen und aufwändig zu entsorgen sind. Ferner ist die Aufarbeitung des erhaltenen Reaktionsgemisches aufgrund des Einsatzes metallkationischer Lewissäuren aufwändig (siehe oben).

L.P. Kyrides, J. Am. Chem. Soc. 59, 1937, Seite 206 bis 208 beschreibt die Herstellung von Phthalsäuredichlorid durch Umsetzung von Phthalsäureanhydrid mit Benzotrichlorid in Gegenwart von Zinkchlorid. Nachteilig an diesem Verfahren ist die Bildung stöchiometrischer Mengen an Benzoylchlorid als Koppelprodukt, welches vom Wertprodukt Phthalsäuredichlorid abzutrennen und aufwändig zu entsorgen ist. Ferner ist die Aufarbeitung des erhaltenen Reaktionsgemisches aufgrund des Einsatzes von Zinkchlorid aufwändig (siehe oben).

Gemeinsamer Nachteil der oben genannten Verfahren ist die erwähnte Bildung stöchiometrischer Mengen an Koppelprodukt, welches im Reaktionsgemisch verbleibt und welches vom Wertprodukt Phthalsäuredichlorid abzutrennen und aufwändig zu entsorgen ist. Beim Einsatz von Thionylchlorid oder Phosgen als Chlorierungsmittel tritt dieser Nachteil nicht auf, da hierbei aus dem Chlorierungsmittel nur gasförmige Nebenprodukte entstehen, nämlich Chlorwasserstoffgas, Schwefeldioxid beziehungsweise Kohlendioxid, welche leicht abzutrennen sind.

So lehrt L.P. Kyrides, J. Am. Chem. Soc. 59, 1937, Seite 206 bis 208 ferner die Herstellung von Phthalsäuredichlorid durch Umsetzung von Phthalsäureanhydrid mit Thionylchlorid in Gegenwart von Zinkchlorid. Das genannte Verfahren weist gegenüber den oben genannten Verfahren zumindest den Vorteil auf, dass das zu verwendende Chlorierungsmittel Thionylchlorid nur gasförmige Nebenprodukte bildet, welche leicht abzutrennen sind. Dennoch besitzt dieses Verfahren entscheidende Nachteile. So ist die erforderliche Reaktionstemperatur mit 220°C sehr hoch und die erzielbare Ausbeute mit 86 % nur mittelmäßig (siehe experimenteller Teil in L.P. Kyrides), auch wenn eine Abschätzung der erzielten Raum-Zeit-Ausbeute (siehe Abschnitt Definitionen) einen respektablen Wert von etwa 73 g/l·h ergibt. Ferner ist die Aufarbeitung des erhaltenen Reaktionsgemisches aufgrund des Einsatzes von Zinkchlorid aufwändig (siehe oben).

US 3,810,940 und DE-A 102 37 579 lehren die Herstellung von Phthalsäuredichlorid durch Umsetzung von Phthalsäureanhydrid mit Phosgen in Gegenwart eines N,N-Dialkyl-formamids und in Gegenwart eines inerten Lösungsmittels. US 3,810,940 lehrt hierbei konkret den Einsatz von N,N-Dimethyl-formamid als Katalysator sowie von Chlorbenzol als Lösungsmittel. Auch diese Verfahren weisen den Vorteil auf, dass das zu verwendende Chlorierungsmittel Phosgen nur gasförmige Nebenprodukte bildet, welche leicht abzutrennen sind. Dennoch zeigen aber diese Verfahren auch

entscheidende Nachteile. So sind die abgeschätzten erzielbaren Raum-Zeit-Ausbeuten mit einem Bereich von 31 bis 52 g/l·h (DE-A 102 37 579 Beispiel 1: etwa 42 g/l·h, Beispiel 2: etwa 31 g/l-h, Beispiel 3: etwa 32 g/l-h; US 3,810,940 Beispiel 4: etwa 52 g/l-h) sehr niedrig und somit absolut unbefriedigend. Ferner werden durch den Einsatz von N,N-Dialkyl-formamiden als Katalysator durch Umsetzung mit Phosgen Amid-Hydrochlorid-Addukte, sogenannte Vilsmeier-Addukte, gebildet, welche ionischer Natur sind und daher bei der nachfolgendnen Destillation zu Problemen infolge Feststoffausfall führen können. Zudem können sich die gebildeten Amid-Hydrochlorid-Addukte bei der nachfolgenden Destillation thermisch zersetzen und zu einer Verunreinigung des Wertprodukts Phthalsäuredichlorid führen.

DE-A 40 06 370 offenbart die allgemeine Lehre zur Synthese von aliphatischen, aromatischen oder araliphatischen Carbonsäurechloriden aus Phosgen und den entsprechenden Carbonsäuren oder Carbonsäureanhydriden in Gegenwart von dialkylierten Carbonsäureamiden und/oder trisubstituierten Phosphanoxiden und/oder sulfiden als Phosgenierungskatalysatoren und insbesondere von Dimethylformamid, Dimethylacetamid und Trioctylphosphinoxid.

Aufgabe der vorliegenden Erfindung war es, ein Verfahren zur Herstellung von Phthalsäuredichlorid hoher Reinheit zu finden, welches die oben genannten Nachteile nicht besitzt, nur leicht zugängliche und technisch gut verfügbare Edukte sowie gegebenenfalls nur leicht zugängliche und technisch gut verfügbare Hilfsstoffe/Katalysatoren erfordert, und die Edukte zur Erhöhung der Raum-Zeit-Ausbeute und zur Ermöglichung einer technisch weniger aufwändigen Isolierung des Wertprodukts Phthalsäuredichlorid keine, überwiegend im Reaktionsgemisch verbleibenden Koppelprodukte bilden. Ferner soll bei dem zu findenden Verfahren das Wertprodukt Phthalsäuredichlorid von eventuell erforderlichen Hilfsstoffen/Katalysatoren sowie von eventuell anfallenden Zwischen- und Nebenprodukten leicht abtrennbar sein, die eventuell erforderlichen Hilfsstoffe/Katalysatoren sowie deren mögliche Umsetzungsprodukte unter den vorliegenden Bedingungen nicht zum Feststoffausfall neigen sowie die Entsorgung der eventuell erforderlichen Hilfsstoffe/Katalysatoren ohne großen Aufwand möglich sein. Des Weiteren soll das zu findenden Verfahren auch unter milden Temperaturen und Drücken zu einem hohen Umsatz an Phthalsäureanhydrid, einer hohen Selektivität zu und einer hohen Ausbeute an Phthalsäuredichlorid führen sowie insbesondere eine hohe Raum-Zeit-Ausbeute aufweisen.

Demgemäß wurde ein Verfahren zur Herstellung von Phthalsäuredichlorid durch Umsetzung von Phthalsäureanhydrid mit einem Chlorierungsmittel (I), ausgewählt aus der Gruppe Thionylchlorid und Phosgen, in Gegenwart eines Katalysators bei einer Temperatur von 80 bis 200°C und einem Druck von 0,01 bis 10 MPa abs, das dadurch gekennzeichnet ist, dass man als Katalysator (II) Triphenylphosphin, Triphenylphosphinoxid oder dessen Gemisch einsetzt.

Das beim erfindungsgemäßen Verfahren als Katalysator (II) einzusetzende Triphenylphosphin, Triphenylphosphinoxid oder dessen Gemisch ist gekennzeichnet durch die gemeinsame Strukturformel (IIa)

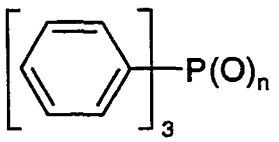

(IIa),

mit n gleich 0 oder 1.

Erfindungsgemäß wurde dabei erkannt, dass andere Phosphine, wie beispielsweise Trialkylphosphine und Trialkylphosphinoxide zu dramatisch niedrigeren Umsätzen, Ausbeuten und Raum-Zeit-Ausbeuten führen (siehe hierzu Vergleichsbeispiel 10).

Die beim erfindungsgemäßen Verfahren einzusetzende Menge an Katalysator (II) beträgt im Allgemeinen 0,05 bis 25 Mol%, bevorzugt 0,1 bis 20 Mol-%, besonders bevorzugt 0,2 bis 10 Mol-% und ganz besonders bevorzugt 0,5 bis 5 Mol%, bezogen auf das eingesetzte Phthalsäureanhydrid.

Als Chlorierungsmittel (1) setzt man beim erfindungsgemäßen Verfahren Thionylchlorid oder Phosgen ein. Das eingesetzte Molverhältnis an Chlorierungsmittel (1) beträgt im Allgemeinen 0,95 bis 10, bevorzugt 0,95 bis 5, besonders bevorzugt 0,98 bis 3 und ganz besonders bevorzugt 1 bis 2, bezogen auf die molare Menge an eingesetztem Phthalsäureanhydrid.

Beim erfindungsgemäßen Verfahren bevorzugt ist der Einsatz von Thionylchlorid als Chlorierungsmittel (1).

Die Zugabe von Phthalsäureanhydrid, des Chlorierungsmittels (I), des Katalysators (II) und eventuell einzusetzender Lösungsmittel kann auf unterschiedliche Art und Weise und in unterschiedlicher Reihenfolge erfolgen.

Das Phthalsäureanhydrid kann beispielsweise aufgeschmolzen oder gelöst in einem inerten Lösungsmittel eingesetzt werden. Ferner ist es möglich, einen Teil oder die gesamte Menge an Katalysator (II) im Phthalsäureanhydrid, gegebenenfalls in Gegenwart eines zusätzlichen inerten Lösungsmittels, zu lösen und auf diese Art und Weise zuzugeben.

Die Zugabe des Chlorierungsmittel (1) erfolgt beim Einsatz von Thionylchlorid in der Regel flüssig und beim Einsatz von Phosgen in der Regel gasförmig. Je nachdem ist es aber auch möglich, Phosgen flüssig zuzugeben. Des

weiteren ist es prinzipiell auch möglich, einen Teil oder die gesamte Menge an Katalysator (II) im Chlorierungsmittel, gegebenenfalls in Gegenwart eines zusätzlichen inerten Lösungsmittels, zu lösen und auf diese Art und Weise zuzugeben. Beim Einsatz von Thionylchlorid ist es bevorzugt, einen Teil des Katalysators (II) darin zu lösen und in dieser Form zum Phthalsäureanhydrid zuzuführen.

Alternativ zu den oben genannten Zugabearten für den Katalysator (II) kann dieser auch separat, gelöst in der erforderlichen Menge an inertem Lösungsmittel, zugegeben werden.

Das erfindungsgemäße Verfahren wird in der Regel halbkontinuierlich oder kontinuierlich betrieben. Bei der halbkontinuierlichen Fahrweise wird üblicherweise Phthalsäureanhydrid in einem geeigneten Reaktionsapparat in aufgeschmolzenem Zustand oder gelöst in einem inerten Lösungsmittel vorgelegt und darin die Gesamtmenge an Katalysator (II) oder zumindest ein Teil davon gelöst. Anschließend gibt man das Chlorierungsmittel (I), welches gegebenenfalls die restliche Menge des Katalysators (II) und gegebenenfalls ein inertes Lösungsmittel enthält, entsprechend dem Reaktionsfortschritt kontinuierlich zu.

Bei der kontinuierlichen Fahrweise werden üblicherweise die Edukte und der Katalysator (II), welche gegebenenfalls in einem inerten Lösungsmittel gelöst sind, gleichzeitig einem geeigneten Reaktionsapparat zugeführt, wobei eine der zugeführten Menge entsprechende Menge gleichzeitig aus dem Reaktionsapparat abgeführt wird.

Als geeignete Reaktionsapparate seien prinzipiell alle Reaktionsapparate genannt, welche für gas/flüssig- oder flüssig/flüssig-Reaktionen geeignet sind, wie beispielsweise Rührkessel.

Unter inerte Lösungsmittel sind Verdünnungsmittel zu verstehen, welche sich unter den gewählten Bedingungen nicht mit den genannten Stoffen chemisch umsetzen. Bevorzugt setzt man aromatische oder aliphatische Kohlenwasserstoffe sowie Phthalsäuredichlorid (Smp. 12°C) ein. Letzteres hat den Vorteil, dass hierduch keine weiteren Fremdstoffe in das Verfahren eingeführt werden. Werden inerte Lösungsmittel eingesetzt, so wählt man bevorzugt solche, welche zur Vermeidung der Siedekühlung einen höheren Siedepunkt als das eingesetzte Chlorierungsmittel (I) besitzen und zudem zur ausreichenden Abtrennbarkeit des Phthalsäuredichlorids bei der destillativen Aufarbeitung einen um bevorzugt mindestens 10°C niedrigeren Siedepunkt, gemessen unter Normaldruck, als Phthalsäureanhydrid besitzen. Alternativ sind jedoch auch inerte Lösungsmittel einsetzbar, welche zur ausreichenden Abtrennbarkeit des Phthalsäuredichlorids einen um bevorzugt mindestens 10°C höheren Siedepunkt, gemessen unter Normaldruck, als Phthalsäuredichlorid besitzen. Als bevorzugte Kohlenwasserstoffe setzt man einfach oder mehrfach substituierte aromatische Kohlenwasserstoffe, wie beispielsweise Toluol, o-, m-, p-Xylol, Ethylbenzol, Chlorbenzol oder o-, m-, p-Dichlorbenzol ein. Darunter ganz besonders bevorzugt sind o-, m- oder p-Xylol, Chlorbenzol, o-, m-, p-Dichlorbenzol oder deren Gemische.

Werden inerte Lösungsmittel bei der Umsetzung eingesetzt, so beträgt deren Menge im Allgemeinen bis zu 2000 Gew.% und bevorzugt bis 1 zu 1000 Gew.%, bezogen auf die Gesamtmenge des vorliegenden Phthalsäureanhydrids und des gebildeten Phthalsäuredichlorids.

Um eine möglichst hohe Raum-Zeit-Ausbeute zu erhalten ist es beim erfindungsmäßen Verfahren bevorzugt, möglichst wenig oder kein Lösungsmittel einzusetzen. So führt man die Umsetzung bevorzugt mit einem Gemisch durch, welches während der gesamten Durchführung der Umsetzung zu ≥ 80 Gew.-%, bevorzugt ≥ 90 Gew.% und besonders bevorzugt zu ≥ 99 Gew.% aus Phthalsäureanhydrid, Chlorierungsmittel (I), Katalysator (II) und aus diesen Stoffen gebildeten Zwischen-, Neben- und Endprodukten besteht. Ganz besonders bevorzugt führt man die Umsetzung in Abwesenheit eines zusätzlichen Lösungsmittels durch.

Unter "Endprodukte" sind die gemäß der Hauptreaktionsgleichungen

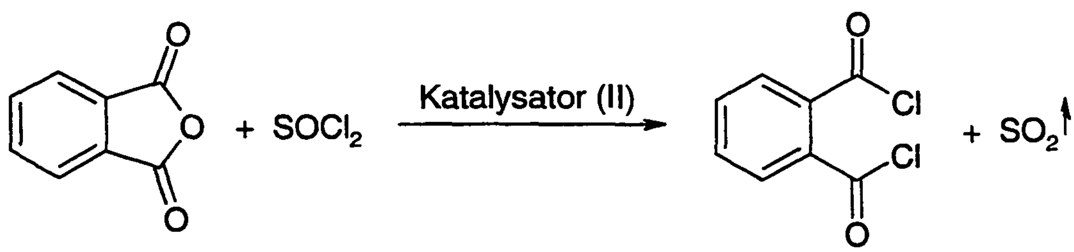

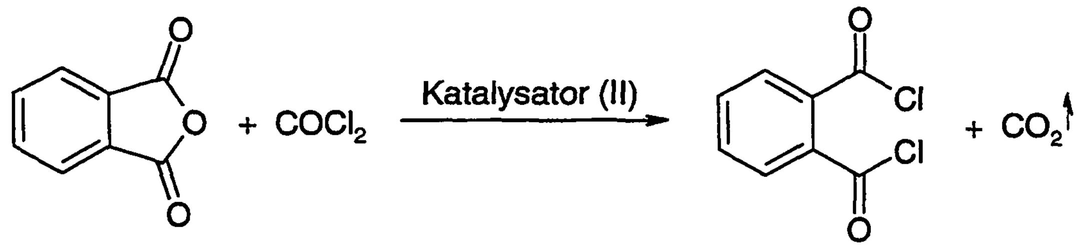


gebildeten Produkte Phthalsäuredichlorid und Schwefeldioxid beziehungsweise Kohlendioxid zu verstehen. Es sei an dieser Stelle betont, dass die Hauptmengen an gebildetem Schwefeldioxid beziehungsweise Kohlendioxid bereits während der Durchführung der Reaktion aus dem flüssigen Reaktionsgemisch gasförmig entweichen.

Unter "Nebenprodukte" sind die Produkte zu verstehen, welche durch Nebenreaktionen gebildet werden. Als Beispiel hierzu sei das 4-Chlorphthalsäuredichlorid genannt. Des Weiteren werden als Nebenprodukte auch die Umsetzungsprodukte des eingesetzten Katalysators (II) angesehen, sofern diese am Ende der erfindungsgemäßen Umsetzung im Reaktionsgemisch vorliegen, beispielsweise Verbindungen, welche durch die Umsetzung von Triphenylphosphin oder Triphenylphosphinoxid mit Thionylchlorid oder Phosgen gebildet werden.

Unter "Zwischenprodukte" sind die während der Umsetzung intermediär auftretenden Produkte zu verstehen.

Das erfindungsgemäße Verfahren führt man bei einem Druck von 0,01 bis 10 MPa abs, bevorzugt von 0,05 bis 5 MPa abs, besonders bevorzugt von 0,09 bis 0,5 MPa abs und ganz besonders bevorzugt von 0,09 bis 0,2 MPa abs durch.

Des Weiteren führt man das erfindungsgemäße Verfahren bei einer Temperatur von 80 bis 200°C, bevorzugt von 100 bis 180°C, besonders bevorzugt von 120 bis 160°C und ganz besonders bevorzugt von 130 bis 160°C durch.

Nachdem die gewünschte Menge an Chlorierungsmittel zugegeben wurde, wird die erhaltene Reaktionslösung in der Regel noch für eine gewisse Zeit, im Allgemeinen 30 Minuten bis 6 Stunden unter den Reaktionsbedingungen zur Nachreaktion belassen. Um überschüssiges Thionylchlorid beziehungsweise Phosgen und dessen Reaktionsprodukte Schwefeldioxid beziehungsweise Kohlendioxid aus der Reaktionslösung zu entfernen beziehungsweise abzureichem, wird anschließend in der Regel unter intensiver Durchmischung Inertgas durchgeleitet ("Strippen").

Der Reaktionsaustrag wird im Allgemeinen mit den bekannten Methoden aufgearbeitet. Vorzugsweise wird das gewünschte Phthalsäuredichlorid durch fraktionierte Destillation isoliert. Das als Katalysator (II) eingesetzte Triphenylphosphin beziehungsweise Triphenylphosphinoxid kann dabei bei Bedarf durch Destillation zurückgewonnen und wieder eingesetzt werden.

Zur Erzielung besonders hoher Produktreinheiten ist es ferner möglich, im destillierten Phthalsäuredichlorid gegebenenfalls gelöstes Phthalsäureanhydrid durch Zugabe eines unpolaren Lösungsmittels, wie beispielsweise Cyclohexan, Petrolether oder Toluol auszufällen und als Feststoff abzutrennen. Das zugegebene Lösungsmittel kann dann anschließend vom Phthalsäuredichlorid wieder abdestilliert werden.

In einer bevorzugten Ausführungsform zur halbkontinuierlichen Herstellung von Phthalsäuredichlorid unter Einsatz von Thionylchlorid gibt man die gewünschte Menge an Phthalsäureanhydrid in einen Rührkessel mit Rückflusskühler und erhitzt dieses auf etwa 130 bis 160°C, so dass eine Phthalsäureanhydrid-Schmelze vorliegt. In diese gibt man unter Rühren etwa die Hälfte des gewünschten Katalysators (II). Alternativ hierzu ist es aber auch möglich, festes Phthalsäureanhydrid und etwa die Hälfte des gewünschten Katalysators (II) vorzulegen und zusammen aufzuschmelzen. Anschließend beginnt man mit der Zugabe einer flüssigen Mischung aus dem Thionylchlorid und dem restlichen Katalysator (II), wobei die Zugabegeschwindigkeit in der Regel derat eingestellt wird, dass das nicht umgesetzte Thionylchlorid schwach unter Rückfluss siedet. Nach Beendigung der Zugabe der Thionylchlorid/Katalysator (II)-Mischung belässt man das Reaktionsgemisch unter weiterem Rühren für etwa 0,5 bis 6 Stunden zur Nachreaktion und strippt anschließend restliches Schwefeldioxid mit Stickstoff heraus. Zuletzt führt man das Reaktionsgemisch einer Destillationskolonne zu, in der man zunächst das überschüssige Thionylchlorid und anschließend, bevorzugt unter Vakuum, das Phthalsäuredichlorid abdestilliert.

In einer anderen bevorzugten Ausführungsform zur halbkontinuierlichen Herstellung von Phthalsäuredichlorid unter Einsatz von Thionylchlorid oder Phosgen gibt man die gewünschte Menge an Phthalsäureanhydrid in einen Rührkessel mit Rückflusskühler und erhitzt dieses auf etwa 130 bis 160°C, so dass eine Phthalsäureanhydrid-Schmelze vorliegt. In diese gibt man unter Rühren den gewünschten Katalysator (II). Alternativ hierzu ist es aber auch möglich, festes Phthalsäureanhydrid und den gewünschten Katalysators (11) vorzulegen und zusammen aufzuschmelzen. Anschließend beginnt man mit der Zugabe Thionylchlorid oder Phosgen, wobei die Zugabegeschwindigkeit in der Regel derat eingestellt wird, dass das nicht umgesetzte Thionylchlorid oder Phosgen schwach unter Rückfluss siedet. Nach Beendigung der Zugabe des Chlorierungsmittels belässt man das Reaktionsgemisch unter weiterem Rühren für etwa 0,5 bis 6 Stunden zur Nachreaktion und strippt anschließend restliches Schwefeldioxid beziehungsweise Kohlendioxid

mit Stickstoff heraus. Zuletzt führt man das Reaktionsgemisch einer Destillationskolonne zu, in der man zunächst das überschüssige Chlorierungsmittel und anschließend, bevorzugt unter Vakuum, das Phthalsäuredichlorid abdestilliert.

Das erfindungsgemäße Verfahren ermöglicht die Herstellung von Phthalsäuredichlorid hoher Reinheit, wobei nur leicht zugängliches und technisch gut verfügbares Phthalsäureanhydrid, Thionylchlorid oder Phosgen als Chlorierungsmittel sowie Triphenylphosphin oder Triphenylphosphinoxid als Katalysator einzusetzen sind und keine, überwiegend im Reaktionsgemisch verbleibende Koppelprodukte gebildet werden. Ferner führt das gefundene Verfahren auch unter milden Temperaturen und Drücken zu einem hohen Umsatz an Phthalsäureanhydrid, einer hohen Selektivität zu und einer hohen Ausbeute an Phthalsäuredichlorid sowie insbesondere zu einer hohe Raum-Zeit-Ausbeute. So können beim erfindungsgmäßen Verfahren Raum-Zeit-Ausbeuten von deutlich über 60 g Phthalsäuredichlorid pro Liter Reaktionsvolumen und Stunde erzielt werden. Des Weiteren ist das Wertprodukt Phthalsäuredichlorid aus dem Reaktionsgemisch leicht abtrennbar, wobei vom Reaktionsgemisch keine Gefahr eines Feststoffausfalls ausgeht. Der bei der anschließenden Produktdestillation im Sumpf verbleibende Katalysator (II) kann daraus bei Bedarf wieder gewonnen oder zusammen mit dem restlichen Sumpfprodukt entsorgt oder thermisch verwertet werden.

Definitionen

Abschätzung der Raum-Zeit-Ausbeute

Da im nächstliegenden Stand der Technik (insbesondere L.P. Kyrides, J. Am. Chem. Soc. 59, 1937, Seite 206 bis 208 und DE-A 102 37 579) bei der Beschreibung der Versuche keine Reaktorgefäßgrößen genannt sind, aber dennoch die Raum-Zeit-Ausbeute im Rahmen der vorliegenden Aufgabenstellung eine zentrale Größe ist, wurden die Raum-Zeit-Ausbeuten abgeschätzt. Um eine Vergleichbarkeit innerhalb der vorliegenden Patentanmeldung zu ermöglichen, wurden sowohl alle Raum-Zeit-Ausbeuten aus dem Stand der Technik als auch jene aus allen erfindungsgemäßen Beispielen und Vergleichsbeispielen nach den gleichen Regeln abgeschätzt. Die Abschätzung erfolgte wie im Folgenden geschildert:

Der Abschätzung liegt die Formel

$$RZA = \frac{m_{Phthalsäuredichlorid}}{V_{Reaktor} \cdot t}$$

zugrunde. $m_{Phthalsäuredichlorid}$ ist dabei die Masse an gebildetem Phthalsäuredichlorid in g, $V_{Reaktor}$ das abgeschätzte Volumen eines für den konkreten Reaktionsansatz technisch sinnvollen Reaktors und t die Reaktionszeit in Stunden, inklusive Nachrührzeit. Zur Abschätzung der Größe des technisch sinnvollen Reaktors wurde davon ausgegangen, dass dieser im Laufe der Reaktion einen maximalem Füllstand von 60 Vol.-% erreicht. Da bei allen Versuchen im nächstliegenden Stand der Technik und in der vorliegenden Patentanmeldung das Chlorierungsagens kontinuierlich zugegeben wurde und somit bereits im Wesentlichen während der Zugabe umgesetzt wurde, wurde davon ausgegangen, dass das maximale Volumen des Reaktionsgemisches jeweils am Versuchsende vorlag. Dieses Volumen wurde aus den Volumina des gebildeten Phthalsäuredichlorids, des verbleibenden Rests zwischen dem eingesetzten Phthalsäureanhydrid und dem gebildeten Phthalsäuredichlorid, dem eingesetzten Katalysator, dem eingesetzten Lösungsmittel und dem in der Lösung verleibenden, nicht-umgesetzten Chlorierungsmittel abgeschätzt. Bei letzterem wurde näherungsweise davon ausgegangen, dass sämtlicher Überschuß an Chlorierungsmittel in der Lösung verblieb und nur die gebildeten Reaktionsprodukte Schwefeldioxid beziehungsweise Kohlendioxid entwichen.

Beispiele

Beispiel 1 (erfindungsgemäß)

Zu einer Mischung aus 74 g (0,5 Mol) Phthalsäureanhydrid und 6,5 g (0,025 Mol) Triphenylphosphin wurde bei 140°C eine Lösung von 6,5 g (0,025 Mol) Triphenylphosphin in 150 g (1,26 Mol) Thionylchlorid binnen 3,5 Stunden zugegeben. Nach Zugabe von 65 mL dieser Lösung fiel die Innentemperatur auf ca. 125°C ab. Nachdem die Gesamtmenge der Lösung zugegeben war, wurde noch 2 Stunden bei 115°C nachgerührt. Anschließend wurden restliches gelöstes Schwefeldioxid mit Stickstoff ausgestrippt. Es wurden 162 g homogener Reaktionsaustrag erhalten, welcher nach Vakuumdestillation 96 g Produkt ergab. Dieses enthielt 94,6 GC-Flächen% Phthalsäuredichlorid (0,447 Mol, entsprechend 89% Ausbeute). Die abgeschätzte Raum-Zeit-Ausbeute betrug etwa 72 g/l.h.

Beispiel 2 (erfindungsgemäß)

Zu einer Mischung aus 74 g (0,5 Mol) Phthalsäureanhydrid und 13 g (0,047 Mol) Triphenylphosphinoxid wurden bei 140°C 122 g (1,03 Mol) Thionylchlorid binnen 4 Stunden zugegeben. Nach Zugabe von 35 mL dieser Lösung fiel die Innentemperatur auf ca. 132°C ab. Nachdem die Gesamtmenge der Lösung zugegeben war, wurde noch 2 Stunden bei 122°C nachgerührt. Anschließend wurden restliches gelöstes Schwefeldioxid mit Stickstoff ausgestrippt. Es wurden 159 g homogener Reaktionsaustrag erhalten, welcher nach Vakuumdestillation 97 g Produkt ergab. Dieses enthielt 94,2 GC-Flächen% Phthalsäuredichlorid (0,45 Mol, entsprechend 90% Ausbeute). Die abgeschätzte Raum-Zeit-Ausbeute betrug etwa 75 g/l·h.

Beispiel 3 (erfindungsgemäß)

Zu einer Mischung aus 74 g (0,5 Mol) Phthalsäureanhydrid und 6,5 g (0,023 Mol) Triphenylphosphinoxid wurde bei 140°C eine Lösung von 6,5 g (0,023 Mol) Triphenylphosphinoxid in 122 g (1,03 Mol) Thionylchlorid binnen 4 Stunden zugegeben. Nach Zugabe von 35 mL fiel die Innentemperatur auf ca. 132°C ab. Nachdem die Gesamtmenge der Lösung zugegeben war, wurde noch 2 Stunden bei 122°C nachgerührt. Anschließend wurden restliches gelöstes Schwefeldioxid mit Stickstoff ausgestrippt. Es wurden 161 g homogener Reaktionsaustrag erhalten, welcher nach Vakuumdestillation 98 g Produkt ergab. Dieses enthielt 94,4 GGFlächen% Phthalsäuredichlorid (0,456 Mol, entsprechend 91 % Ausbeute). Die abgeschätzte Raum-Zeit-Ausbeute betrug etwa 76 g/l.h.

Beispiel 4 (erfindungsgemäß)

Zu einer Mischung aus 74 g (0,5 Mol) Phthalsäureanhydrid und 2,0 g (0,007 Mol) Triphenylphosphinoxid wurde bei 142°C eine Lösung von 4,5 g (0,016 Mol) Triphenylphosphinoxid in 122 g (1,03 Mol) Thionylchlorid binnen 5 Stunden zugegeben. Nach Zugabe von 40 mL fiel die Innentemperatur auf ca. 127°C ab. Nachdem die Gesamtmenge der Lösung zugegeben war, wurde noch 2 Stunden bei 112°C nachgerührt. Anschließend wurden restliches gelöstes Schwefeldioxid mit Stickstoff ausgestrippt. Der erhaltene homogene Reaktionsaustrag (162 g) wurde zunächst bei 200 hPa abs (200 mbar abs) und 35°C von überschüssigem Thionylchlorid befreit und anschließend bei 9 hPa abs (9 mbar abs) und 137°C destilliert. Das Destillat (97,7 g), aus dem beim Stehenlassen wenige Kristalle Phthalsäureanhydrid ausfielen, wurde mit 50 mL Cyclohexan versetzt. Vom Gemisch wurden 5,3 g ausgefallenes Phthalsäureanhydrid abgesaugt und anschließend das Cyclohexan abdestilliert. Es verblieben 92,0 g Produkt, welches 96,9 GC-Flächen-% Phthalsäuredichlorid enthielt (0,44 Mol, entsprechend 88% Ausbeute). Die abgeschätzte Raum-Zeit-Ausbeute betrug etwa 66 g/l-h.

Beispiel 5 (erfindungsgemäß)

Zu einer Mischung aus 650 g (4,4 Mol) Phthalsäureanhydrid und 30 g (0,11 Mol) Triphenylphosphinoxid wurde bei 140°C eine Lösung von 30 g (0,11 Mol) Triphenylphosphinoxid in 720 g (6,05 Mol) Thionylchlorid binnen 8 Stunden zugegeben. Nach Zugabe von 120 mL dieser Lösung fiel die Innentemperatur auf ca. 132°C ab. Nachdem die Gesamtmenge der Lösung zugegeben war, wurde noch 2 Stunden bei 122°C nachgerührt. Anschließend wurden restliches gelöstes Schwefeldioxid mit Stickstoff ausgestrippt. Es wurden 1249 g homogener Reaktionsaustrag erhalten, welcher nach Vakuumdestillation 826 g Produkt ergab. Dieses enthielt 94,2 GC-Flächen% Phthalsäuredichlorid (3,8 Mol, entsprechend 87% Ausbeute). Die abgeschätzte Raum-Zeit-Ausbeute betrug etwa 58 g/l-h.

Die Beispiele 1 bis 5 zeigen, dass beim erfindungsgemäßen Einsatz von Thionylchlorid als Chlorierungsmittel und von Triphenylphosphin beziehungsweise Triphenylphosphinoxid als Katalysator Raum-Zeit-Ausbeuten im Bereich von 58 bis 76 g/l.h zu erreichen sind.

Beispiel 6 (erfindungsgemäß)

Zu einer Mischung aus 224 g (1,5 Mol) Phthalsäureanhydrid und 5,3 g (0,019 Mol) Triphenylphosphinoxid wurden bei 135°C 181 g (1,82 Mol) Phosgen binnen 5 Stunden zugegeben. Nach beendeter Zugabe wurde noch 0,5 Stunden bei 113°C nachgerührt. Anschließend wurden Phosgen und gelöstes Kohlendioxid bei 60°C mit Stickstoff ausgestrippt. Es wurden 308 g homogener Reaktionsaustrag erhalten, welcher nach Vakuum-Destillation (1 hPa abs (1 mbar abs)) 300 g Produkt ergab. Dieses enthielt 98,5 GC-Flächen-% Phthalsäuredichlorid (1,48 Mol, entsprechend 98,7% Ausbeute). Die abgeschätzte Raum-Zeit-Ausbeute betrug etwa 131 g/l·h.

Beispiel 6 zeigt, dass beim erfindungsgemäßen Einsatz von Phosgen als Chlorierungsmittel und von Triphenylphosphinoxid als Katalysator Raum-Zeit-Ausbeuten von deutlich oberhalb 100 g/l.h zu erreichen sind. Es sei an dieser Stelle erneut betont, dass die Raum-Zeit-Ausbeuten nach dem Stand der Technik (US 3,810,940 und DE-A 102 37 579) bei der Phosgenierung von Phthalsäureanhydrid in Gegenwart von N,N-Dimethylformamid als Katalysator mit

31 bis 52 g/l·h nur einen Bruchteil der Raum-Zeit-Ausbeuten des erfindungsgemäßen Verfahrens betragen.

Beispiel 7 (erfindungsgemäß)

Zu einer Mischung aus 224 g (1,5 Mol) Phthalsäureanhydrid und 5,3 g (0,019 Mol) Triphenylphosphinoxid in 306 g Chlorbenzol (275 mL) wurden bei 126°C 164 g (1,66 Mol) Phosgen binnen 4 Stunden zugegeben. Nach beendeter Zugabe wurde noch 0,5 Stunden bei 120°C nachgerührt. Anschließend wurden Phosgen und gelöstes Kohlendioxid bei 60°C mit Stickstoff ausgestrippt. Es wurden 609 g homogener Reaktionsaustrag erhalten, welcher gaschromatographisch analysiert wurde. Nach rechnerischem Abzug des Lösungsmittels Chlorbenzol und des eingesetzten Katalysators enthielt der Reaktionsaustrag 97,9 GC-Flächen-% Phthalsäuredichlorid neben 2,1 GC-Flächen% nicht-umgesetztes Phthalsäureanhydrid, was einer Ausbeute von 97,9 % entspricht. Die abgeschätzte Raum-Zeit-Ausbeute betrug etwa 79 g/l.h.

Beispiel 8 (Vergleichsbeispiel)

Zu einer Mischung aus 224 g (1,5 Mol) Phthalsäureanhydrid und 5,3 g (0,019 Mol) Triphenylphosphinoxid in 306 g Chlorbenzol (275 mL) wurden bei 70°C 72 g (0,73 Mol) Phosgen eingeleitet. Es war starker Rückfluss zu beobachten, was auf die fehlende oder nur sehr unzureichende Umsetzung des Phosgens hinwies. Die Innentemperatur fiel dabei auf etwa 50°C. Der Versuch wurde abgebrochen und das Reaktionsgemisch gaschromatographisch analysiert. Es konnte keine Bildung nennenswerter Mengen an Phthalsäuredichlorid nachgewiesen werden, was einer Raum-Zeit-Ausbeute von 0 g/l·h entspricht.

Beispiel 8 zeigt, dass eine Reaktionstemperatur von 70°C für die Umsetzung unzureichend ist. Demgegenüber konnte in Beispiel 7 bei einer Reaktionstemperatur von 126°C selbst im Gegenwart eines Lösungsmittels ein sehr hoher Umsatz erzielt werden.

Beispiel 9 (Vergleichsbeispiel)

Zu einer Mischung aus 224.g (1,5 Mol) Phthalsäureanhydrid und 1,4 g (0,019 Mol) N,N-Dimethylformamid in 306 g Chlorbenzol (275 mL) wurden bei 70°C gemäß Example IV von US 3,810,940 159 g (1,61 Mol) Phosgen binnen 5 Stunden zugegeben. Nach beendeter Zugabe wurde noch 1 Stunde bei 70°C nachgerührt. Anschließend wurden Phosgen und gelöstes Kohlendioxid bei 60°C mit Stickstoff ausgestrippt. Es wurden 560 g zum Teil kristalliner Reaktionsaustrag erhalten, welcher gaschromatographisch analysiert wurde. Nach rechnerischem Abzug des Lösungsmittels Chlorbenzol und des eingesetzten Katalysators enthielt der Reaktionsaustrag 62,2 GC-Flächen% Phthalsäuredichlorid neben 37,6 GC-Flächen-% nicht-umgesetztes Phthalsäureanhydrid, was einer Ausbeute von nur 62,2 % entspricht. Die abgeschätzte Raum-Zeit-Ausbeute betrug nur etwa 41 g/l·h.

Beispiel 9 zeigt klar, dass die in US 3,810,940 gelehrten Verfahrensbedingungen einschließlich des Katalysators zur Erreichung einer hohen Ausbeute und einer hohen Raum-Zeit-Ausbeute absolut ungeeignet sind.

Beispiel 10 (Vergleichsbeispiel)

Zu einer Mischung aus 450 g (3,0 Mol) Phthalsäureanhydrid und 26 g (0,075 Mol) Cyanex® 923 (Gemisch verschiedener Tri-$C_6$- bis $C_8$-alkylphosphinoxide der Firma Cytec Industries) wurde bei 140°C eine Lösung von 26 g (0,075 Mol) Cyanex® 923 in 603 g (5,1 Mol) Thionylchlorid binnen 7 Stunden zugegeben. Nach beendeter Zugabe wurde noch 2 Stunden bei 140°C nachgerührt. Anschließend wurden gelöstes Schwefeldioxid bei 100°C mit Stickstoff ausgestrippt. Der Reaktionsaustrag, welcher zum Teil kristallin war, wurde gaschromatographisch analysiert. Nach rechnerischem Abzug des eingesetzten Katalysators enthielt der Reaktionsaustrag nur 19 GC-Flächen% Phthalsäuredichlorid neben 81 GC-Flächen% nicht-umgesetztes Phthalsäureanhydrid, was einer Ausbeute von nur 19% entspricht. Die abgeschätzte Raum-Zeit-Ausbeute betrug gerade einmal etwa 16 g/l-h.

Das Vergleichsbeispiel 10 zeigt, dass es beim erfindungsmäßen Verfahren signfikant auf die Art des eingesetzten Katalysators ankommt und beispielsweise ein Tri-$C_6$- bis $C_8$-alkylphosphinoxid gegenüber Triphenylphoshinoxid zu dramatisch schlechteren Ergebnissen führt.

**Patentansprüche**

1. Verfahren zur Herstellung von Phthalsäuredichlorid durch Umsetzung von Phthalsäureanhydrid mit einem Chlorierungsmittel (I), ausgewählt aus der Gruppe Thionylchlorid und Phosgen, in Gegenwart eines Katalysators bei einer Temperatur von 80 bis 200°C und einem Druck von 0,01 bis 10 MPa abs, **dadurch gekennzeichnet, dass** man als Katalysator (II) Triphenylphosphin, Triphenylphosphinoxid oder dessen Gemisch einsetzt.

2. Verfahren nach Anspruch 1, **dadurch gekennzeichnet, dass** man 0,1 bis 20 Mol-% Katalysator (II), bezogen auf das eingesetzte Phthalsäureanhydrid, einsetzt.

3. Verfahren nach den Ansprüchen 1 bis 2, **dadurch gekennzeichnet, dass** man das Chlorierungsmittel (I) in einem Molverhältnis von 0,95 bis 10, bezogen auf die molare Menge an eingesetztem Phthalsäureanhydrid, einsetzt.

4. Verfahren nach den Ansprüchen 1 bis 3, **dadurch gekennzeichnet, dass** man als Chlorierungsmittel (I) Thionylchlorid einsetzt.

5. Verfahren nach den Ansprüchen 1 bis 4, **dadurch gekennzeichnet, dass** man die Umsetzung mit einem Gemisch durchführt, welches während der gesamten Durchführung der Umsetzung zu ≥ 80 Gew.% aus Phthalsäureanhydrid, Chlorierungsmittel (1), Katalysator (11) und aus diesen Stoffen gebildeten Zwischen-, Neben- und Endprodukten besteht.

6. Verfahren nach den Ansprüchen 1 bis 4, **dadurch gekennzeichnet, dass** man die Umsetzung mit einem Gemisch durchführt, welches während der gesamten Durchführung der Umsetzung zu ≥ 99 Gew.-% aus Phthalsäureanhydrid, Chlorierungsmittel (I), Katalysator (II) und aus diesen Stoffen gebildeten Zwischen-, Neben- und Endprodukten besteht.

7. Verfahren nach den Ansprüchen 1 bis 4, **dadurch gekennzeichnet, dass** man die Umsetzung in Abwesenheit eines zusätzlichen Lösungsmittels durchführt.

8. Verfahren nach den Ansprüchen 1 bis 7, **dadurch gekennzeichnet, dass** man die Umsetzung bei einem Druck von 0,05 bis 5 MPa abs durchführt.

9. Verfahren nach den Ansprüchen 1 bis 8, **dadurch gekennzeichnet, dass** man die Umsetzung bei einer Temperatur von 120 bis 160°C durchführt.

## Claims

1. A process for preparing phthaloyl chloride by reacting phthalic anhydride with a chlorinating agent (I) selected from the group of thionyl chloride and phosgene in the presence of a catalyst at a temperature of from 80 to 200°C and a pressure of from 0.01 to 10 MPa abs, which comprises using as the catalyst (II) triphenylphosphine, triphenylphosphine oxide or a mixture thereof.

2. The process according to claim 1, wherein from 0.1 to 20 mol% of catalyst (II) based on the phthalic anhydride used are used.

3. Process according to claims 1 and 2, wherein the chlorinating agent (I) is used in a molar ratio of from 0.95 to 10 based on the molar amount of phthalic anhydride used.

4. The process according to claims 1 to 3, wherein the chlorinating agent (I) used is thionyl chloride.

5. The process according to claims 1 to 4, wherein the reaction is carried out with a mixture which consists, during the entire performance of the reaction, to an extent of ≥ 80% by weight of phthalic anhydride, chlorinating agent (I), catalyst (II) and intermediates, by-products and end products formed from these substances.

6. The process according to claims 1 to 4, wherein the reaction is carried out with a mixture which consists, during the entire performance of the reaction, to an extent of > 99% by weight of phthalic anhydride, chlorinating agent (I), catalyst (II) and intermediates, by-products and end products formed from these substances.

7. The process according to claims 1 to 4, wherein the reaction is carried out in the absence of an additional solvent.

8. The process according to claims 1 to 7, wherein the reaction is carried out at a pressure of from 0.05 to 5 MPa abs.

9. The process according to claims 1 to 8, wherein the reaction is carried out at a temperature of from 120 to 160°C.

## Revendications

1. Procédé de préparation de dichlorure d'acide phtalique par réaction d'anhydride d'acide phtalique avec un agent de chloration (I), sélectionné parmi le groupe chlorure de thionyle et phosgène, en présence d'un catalyseur, à une température de 80 à 200 °C et une pression de 0,01 à 10 MPa absolu, **caractérisé en ce que** de la triphénylphosphine, de l'oxyde de triphénylphosphine ou leur mélange est utilisé(e) en tant que catalyseur (II).

2. Procédé selon la revendication 1, **caractérisé en ce que** 0,1 à 20 % en moles de catalyseur (II), par rapport à l'anhydride d'acide phtalique utilisé, est utilisé.

3. Procédé selon l'une quelconque des revendications 1 à 2, **caractérisé en ce que** l'agent de chloration (I) est utilisé en un rapport molaire de 0,95 à 10, par rapport à la quantité molaire d'anhydride d'acide phtalique utilisé.

4. Procédé selon l'une quelconque des revendications 1 à 3, **caractérisé en ce que** du chlorure de thionyle est utilisé en tant qu'agent de chloration (I).

5. Procédé selon l'une quelconque des revendications 1 à 4, **caractérisé en ce que** la réaction est mise en oeuvre avec un mélange qui, pendant la totalité de la mise en oeuvre de la réaction, est constitué d'au minimum 80 % en poids d'anhydride d'acide phtalique, d'agent de chloration (I), de catalyseur (II) et de produits intermédiaires, de sous-produits et de produits finaux formés à partir de ces substances.

6. Procédé selon l'une quelconque des revendications 1 à 4, **caractérisé en ce que** la réaction est mise en oeuvre avec un mélange qui, pendant la totalité de la mise en oeuvre de la réaction, est constitué d'au minimum 99 % en poids d'anhydride d'acide phtalique, d'agent de chloration (I), de catalyseur (II) et de produits intermédiaires, de sous-produits et de produits finaux formés à partir de ces substances.

7. Procédé selon l'une quelconque des revendications 1 à 4, **caractérisé en ce que** la réaction est mise en oeuvre en l'absence d'un solvant supplémentaire.

8. Procédé selon l'une quelconque des revendications 1 à 7, **caractérisé en ce que** la réaction est mise en oeuvre à une pression de 0,05 à 5 MPa absolu.

9. Procédé selon l'une quelconque des revendications 1 à 8, **caractérisé en ce que** la réaction est mise en oeuvre à une température de 120 à 160 °C.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 1464463 A
- US 2051096 A
- DE 2036171 A
- US 3810940 A
- DE 10237579 A
- DE 4006370 A


### In der Beschreibung aufgeführte Nicht-Patentliteratur


- **O. GRÄBE.** *Liebigs Ann.,* vol. 1887, 318-337
- **S. WOLFE et al.** *Canadian Journal of Chemistry,* 1970, vol. 48, 3566-3571
- **S. WOLFE et al.** *Canadian Journal of Chemistry,* 1970, vol. 48, 3570
- **L.P. KYRIDES.** *J. Am. Chem. Soc.,* 1937, vol. 59, 206-208